# EUROPÄISCHE PATENTANMELDUNG

(11) **EP 2 939 650 A1**
(43) Veröffentlichungstag der Anmeldung: **04.11.2015**
(21) Anmeldenummer: 14166374.0
(22) Anmeldetag: 29.04.2014
(51) Int. Cl.: A61K 8/02, A61K 9/70, A61L 15/00, A61K 31/436, A61K 31/573, A61K 36/00

(54) **Pflaster zur Behandlung von Dermatitis**

(71) Anmelder: LTS LOHMANN Therapie-Systeme AG, 56626 Andernach (DE)
(72) Erfinder: Schnitzler, Iris, 53127 Bonn (DE); Maichle, Thomas, 41470 Neuss (DE)
(74) Vertreter: Schweitzer, Klaus

(57) **Zusammenfassung**

Beschrieben ist die Verwendung eines für Wasserdampf durchlässigen Hautpflasters, das ein Hydrogel, ein Feuchthaltemittel und mindestens einen pharmazeutisch und/oder kosmetisch wirksamen Stoff enthält, zur Behandlung von entzündlichen Erkrankungen der Haut, die in den Gruppen L20 bis L30 der ICD-10 (WHO-Version 2006) aufgeführt sind, insbesondere zur Behandlung von Neurodermitis. Das Pflaster wirkt dabei auch durch ein Kühleffekt.

## Beschreibung

Gegenstand der Erfindung ist ein Pflaster zur Behandlung von Dermatitis, insbesondere von Neurodermitis.

Im Stand der Technik sind bereits zahlreiche Möglichkeiten zur Behandlung von Neurodermitis beschrieben. Neben den medikamentösen Therapien zählen dazu auch die Vermeidung von bestimmten Nahrungsmitteln und die Vermeidung von äußeren Reizen und Stress. Eine ursächliche Behandlung der Neurodermitis ist jedoch bis heute nicht gelungen.

Häufig wird die Dermatitis durch eine lokale Therapie behandelt, d.h. durch eine äußerliche Behandlung der betroffenen Hautregionen. Sie zielt darauf ab, die entzündlichen Reaktionen der Haut zu vermindern und den Juckreiz zu lindern. Von Dermatitis betroffene Hautpartien sind zudem vielfach zusätzlich bakteriell infiziert, häufig durch Staphylococcus aureus-Bakterien. Durch entsprechende antibakterielle Therapiemaßnahmen lassen sich diese Infektionen behandeln.

Für die topische Anwendung steht ein breites Spektrum an Wirkstoffen zur Verfügung. Dazu zählen Antibiotika, Cortison, Gerbstoffe, Harnstoff-Präparate, Teerpräparate und Schieferöle sowie topische Immunmodulatoren aus der Klasse der Makrolide. Angewendet werden diese Wirkstoffe in Form von Salben, Cremes, Lotionen oder Lösungen. Die Wirkungsweise ist dazu sehr unterschiedlich:

Antibiotika wirken antibakteriell gegen den Juckreiz auslösende Bakterien. Der Erfolg ist jedoch häufig nur kurzfristig. Sobald die betroffenen Hautregionen von neuen Bakterien besiedelt sind, beginnen die Entzündung und der Juckreiz von neuem.

Cortison wirkt entzündungshemmend, hat aber den Nachteil, dass es nur für kurze Zeit bei aktuellen Neurodermitis-Schüben angewendet werden kann. Eine langfristige Anwendung von Cortison führt zu einer Verdünnung der Haut, zu einer starken Behaarung, zu Dehnungsstreifen sowie zu einer partiellen Unterdrückung des lokalen Immunsystems.

Gerbstoffe wirken entzündungshemmend und leicht juckreizstillend. Sie fördern den Heilungsprozess und regulieren den Wasserhaushalt der Haut. Gerbstoffhaltige Zubereitungen sind jedoch nicht geruchsneutral.

Harnstoff-Präparate bewirken eine Verminderung des Juckreizes, sind keimabtötend und feuchtigkeitserhaltend.

Teerpräparate und Schieferöle wirken entzündungshemmend und lindern den Juckreiz. Ihr Geruch und die intensive Färbung schränken die Verwendung im Alltag jedoch stark ein.

Topische Immunmodulatoren, insbesondere Calcineurin-Inhibitoren auf Basis von Makroliden, stellen das Gleichgewicht in der Immunabwehr der Haut wieder her, was zum Rückgang von Entzündungen, Rötungen und Juckreiz führt. Eine entsprechende Salbe, die den Wirkstoff Tacrolimus enthält, ist kommerziell erhältlich unter der Bezeichnung ®Protopic. Bekannt ist auch eine Creme mit dem Wirkstoff Pimecrolimus (erhältlich unter der Bezeichnung ®Elidel bzw. ®Douglan), die die Bildung von speziellen Allergenen in den Leukozyten hemmt.

Für die topische Anwendung kommen ferner in Frage:
- Hamamelis virginiana-Extrakt in Form von Salben oder Cremes: Der Extrakt wirkt beruhigend auf die Haut, keimabtötend und entzündungshemmend und ist daher zur Behandlung von Neurodermitis besonders geeignet;
- Kamillen-Extrakt und -Salbe: Die Wirkung von Kamille besteht in erster Linie in einer Entzündungshemmung, ggf. auch keimabtötend.
- Dulcamarae stipides: Ein Präparat aus den Stängeln eines Nachtschattengewächses. Es wirkt entzündungshemmend, dämpft allergische Reaktionen und stillt den Juckreiz.
- Echinaceae: Es wird der Press-Saft des frisch blühenden Echinacea-Krauts verwendet. Der Saft hat zahlreiche Effekte auf das Immunsystem. Er unterstützt es bei der Vernichtung von Bakterien und Viren wie auch von Tumorzellen und er stimuliert das Immunsystem in mehreren Bereichen. Auch weist er selbst eine antivirale Wirkung auf.

Zur Linderung des Juckreizes stehen zudem äußerlich anzuwendende Präparate mit einer kühlenden Wirkung zur Verfügung. Meist enthalten sie Substanzen, wie Menthol, Campher oder Polidocanol, die - zu Lotionen verarbeitet - auf die Haut aufgetragen werden und beim Verdunsten kühlend wirken. Eine langfristige Anwendung dieser Präparate führt jedoch zu einem Austrocknen der Haut. Beim Auftragen der Lotionen auf bereits geschädigte oder aufgekratzte Hautregionen kann zudem ein unangenehm brennendes Gefühl entstehen.

Zur Pflege trockener Haut werden allgemein Mittel verwendet, die den Feuchtigkeitsverlust ausgleichen und so die Oberhaut vor Austrocknung schützen. Üblicherweise sind dies Wasser-in-Öl-Emulsionen, die der Haut Feuchtigkeit und Fett zuführen und die Epidermis-Barriere stabilisieren sollen.

Linderung bewirken auch Cremes, Gele oder Lotionen mit Aloe vera, Schwarzkümmelöl und, in neuerer Zeit, auch Argan-Hautöle (Argan-Öl enthält mehr als 80 Gew.-% ein- und mehrfach ungesättigte Fettsäuren, α- und γ-Tocopherole (Vitamin E), Linolsäure und Phytosterole, die für ihre antioxidative Wirkung bekannt sind. Die genannten Stoffe wirken kosmetisch und/oder pharmazeutisch.

Der Nachteil von pharmazeutischen bzw. kosmetischen Cremes, Gelen und Lotionen ist allerdings, dass diese den Juckreiz nur für kurze Zeit stillen. Bei Gelen beträgt der Kühleffekt in der Regel nur wenige Minuten.

Zubereitungen mit einem hohen Gehalt an öligen Komponenten ziehen relativ schnell in die Haut ein, können jedoch auch von Textilien (Bekleidung, Bettwäsche) aufgesogen werden. Generell werden bei der Entwicklung einer Creme Fett und Feuchtigkeit so kombiniert, dass sich die Creme leicht auf der Haut verteilen lässt und schnell einzieht, ohne einen Fettfilm zu hinterlassen.

Bekannt und kommerziell erhältlich sind zudem Pflaster (Patches) zur Behandlung von Cellulitis, die eine Mischung aus einem Hydrogel (Polyacrylat), verschiedenen Wirkstoffen (Coffein, Grüner-Tee-Extrakt, Schachtelhalm-Extrakt, Bitterorangen-Extrakt und Minzöl) und Feuchthaltemitteln (Butylenglykol und Ethanol) enthalten. Die Patches umfassen zudem eine Rückschicht. Sie haben eine kühlende Wirkung auf der Haut.

Es bestand daher nach wie vor die Aufgabe, ein Erzeugnis zur Verfügung zu stellen, das zur Behandlung von Dermatitis, insbesondere von Neurodermatitis, geeignet ist, ohne dabei die oben geschilderten Nachteile aufzuweisen. Das Erzeugnis soll einer Hauttrockenheit verhindern und den Juckreiz lindern. Es soll insbesondere eine kühlende und eine feuchthaltende Wirkung aufweisen. Es soll einfach herzustellen und einfach anwendbar sein.

Gelöst wurde die Aufgabe mit einem Hautpflaster, das ein Hydrogel, mindestens ein Feuchthaltemittel und mindestens einen pharmazeutisch und/oder kosmetisch wirksamen Stoff enthält, der gegen atopische Dermatitis wirksam ist.

Gegenstand der Erfindung ist demgemäß die Verwendung eines für Wasserdampf durchlässigen Hautpflasters, das ein Hydrogel, ein Feuchthaltemittel und mindestens einen pharmazeutisch und/oder kosmetisch wirksamen Stoff enthält, zur Behandlung von entzündlichen Erkrankungen der Haut, die in den Gruppen L20 bis L30 der ICD-10 (WHO-Version 2006) aufgeführt sind, also Atopisches [endogenes] Ekzem (L20), Seborrhoisches Ekzem (L21), Windeldermatitis (L22), Allergische Kontaktdermatitis (L23), Toxische Kontaktdermatitis (L24), nicht näher bezeichnete Kontaktdermatitis (L25), Exfoliative Dermatitis (L26), Dermatitis durch oral, enteral oder parenteral aufgenommene Substanzen (L27), Lichen simplex chronicus und Prurigo (L28), Pruritus (L29) und Sonstige Dermatitis (L39). Gegenstand der Erfindung ist insbesondere die Verwendung eines solchen Pflasters zur Behandlung der Neurodermitis.

Als Dermatitis wird dabei eine entzündliche Reaktion der Haut bezeichnet, die insbesondere die Lederhaut (Dermis) erfasst. Synonym zu Dermatitis wird der Begriff "Ekzem" verwendet. In der Klassifikation nach ICD-10 (WHO-Version 2006) sind diese Erkrankungen in den Gruppen L20 bis L30 aufgeführt. Im Vordergrund dieser Erkrankungen steht eine Intoleranz-Reaktion.

Die Neurodermitis (ICD-10: L20), auch atopische Dermatitis, atopisches Ekzem oder endogenes Ekzem genannt, ist eine in den Industriestaaten relativ weit verbreitete Krankheit. Häufig leiden die Betroffenen auch unter einer Allergie. Die Neurodermitis ist eine heftig juckende, allergisch bedingte Hauterkrankung, die mit geröteten Hautstellen, Schuppung und Bläschenbildung beginnt. Später entwickeln sich an den Beugeseiten der Extremitäten Hautverdickungen und eine vergröberte Haut mit leicht dunklerer Pigmentierung. Die Krankheit hat häufig einen chronischen Verlauf und wird als sehr belastend empfunden. Quälender Juckreiz kann eine starke Beeinträchtigung der Lebensqualität für Neurodermitiker bedeuten.

Auch wenn die Ursache der Neurodermitis bislang nicht zweifelsfrei geklärt ist, wird davon ausgegangen, dass sie sowohl von genetischen Faktoren als auch von Umwelteinflüssen ausgelöst wird. Es wird vermutet, dass die Betroffenen aufgrund genetischer Veranlagung stärker auf bestimmte Umwelteinflüsse reagieren als andere.

Das erfindungsgemäße Pflaster ist jedoch nicht nur zur Behandlung der Neurodermitis geeignet, sondern zur Behandlung aller Krankheiten, die in den Gruppen L20 bis L30 der ICD-10 (WHO-Version 2006) genannt sind.

Es besitzt gute Klebeeigenschaften auf der Haut, insbesondere auch auf den von den Symptomen der Neurodermitis betroffenen Hautregionen.

Die Hydrogel enthaltende Schicht hat allgemein ein Gewicht von 500 bis 1500 g/m², bevorzugt 600 bis 1400 g/m², besonders bevorzugt 800 bis 1200 g/m², d.h. sie ist relativ dick. Sie liegt direkt auf den zu behandelnden Hautpartien auf, ohne irgendeine Klebeschicht.

Der Schälwiderstand beim Abschälen des erfindungsgemäßen Pflasters von der Haut liegt bevorzugt im Bereich von 0,5 bis 4,0 N/25 mm beim 180° Peel-Adhesion-Test.

Das Hydrogel ist ein filmbildendes Polymer. Die Polymerfilme mit einem Schichtgewicht in dem genannten Bereich sind selbsttragend und können auch ohne Rückschicht verwendet werden.

Die Hydrogele selbst bestehen aus hydrophilen und in Wasser quellbaren natürlichen und/oder synthetischen Polymeren. Zu nennen sind insbesondere synthetische Polymere, wie Polyvinylalkohol, teilverseifte Polyvinylacetate, Polyethylenoxide, Poly(hydroxyalkyl(meth)acrylate) und Polyvinylpyrrolidon. Geeignete natürliche Polymere sind beispielsweise Carrageenan, Kollagen, Gelatine, Agar-Agar, Alginat und Chitosan. In einem Hydrogel sind Polymerketten mit hydrophilen funktionellen Gruppen in der Regel dreidimensional vernetzt, so dass sie eine schnittfeste Struktur bilden. Die funktionellen Gruppen sind insbesondere Carboxylgruppen, (primäre) Amidgruppen und/oder Sulfonylgruppen. Die Hydrogele sollten nur gering oder gar nicht wasserlöslich sein.

Feuchthaltemittel, die in der vorliegenden Erfindung eingesetzt werden können sind aufgeführt in "Kosmetika - Inhaltsstoffe - Funktionen" (Industrieverband Körperpflege und Waschmittel e. V. (IKW), http://www.ikw.org, 2. Auflage 2005, Rindt GmbH & Co. KG, Fulda). Verwendbar sind die mit der Nummer "20" gekennzeichneten Stoffe.

Bevorzugt sind die Feuchthaltemittel ausgewählt aus Glycerin, Glykolderivaten wie Butylenglykol, PEG, Sorbit, Harnstoff, Cyclomethicone, Dimethicone, Hyaluronsäure/Milchsäure, Aminosäuren und hydrolysierten Proteinen und Zucker.

Der Anteil des Feuchthaltemittels am Gesamtgewicht der Hydrogel-enthaltenden Schicht beträgt allgemein von 0 bis 25 Gew.-%, vorzugsweise 3 bis 20 Gew.-%, bes. bevorzugt: 10 bis 15 Gew.-%.

Das gegen die genannten Hauterkrankungen pharmazeutisch wirksame Mittel ist insbesondere ein Calcineurin-Inhibitor auf Basis von Makroliden, wie Tacrolimus, Pimecrolimus. Auch Cortison kann in pharmazeutisch wirksamer Menge eingesetzt werden. Pharmazeutisch und/oder kosmetisch wirksam sind auch Pflanzenextrakte und pflanzliche Präparate aus Nachtschattengewächsen, aus Schachtelhalm (Equisetum spp.), Aloe vera, Minze, Bitterorangen, Echinacea, Kamille, Hamamelis virginiana, Schwarzkümmel (Nigella sativa), Viola tricolor, Viola avensis, Johanniskraut (hypericum perforatum), Nachtkerze (oenothera biennis), Calendula (dulcamaris solanis).

In einer bevorzugten Ausführungsform weist das erfindungsgemäße Hautpflaster neben der Hydrogel enthaltenden Schicht eine Rückschicht auf, die ausreichend durchlässig für Wasserdampf ist, um einerseits den kühlenden Effekt zu erhalten und zu regulieren, andererseits die Hydrogel enthaltende Schicht von außen zu schützen. Die Rückschicht besteht vorzugsweise aus einem Vliesstoff, der auf die Hydrogelschicht laminiert ist. Der Vliesstoff besteht beispielsweise aus Viskosefasern oder einem Gemisch von Viskose- und Polyesterfasern. Er ist vorzugsweise vernadelt, beispielsweise durch Wasserstrahlvernadelung. Sein Gewicht beträgt allgemein etwa 70 bis 120 g/m². Das Laminieren erfolgt zweckmäßig unter Wärmeeinwirkung, insbesondere bei etwa 80 bis 90 °C. Die Rückschicht kann ebenso gut aus einem anderen flächenförmigen Fasermaterial, beispielsweise einem Gewebe oder Gewirke, oder aus einer wasserdampfdurchlässigen Folie, beispielspielsweise einer Ethylen/Vinylacetat-Folie, bestehen.

Bei der Herstellung der erfindungsgemäßen Hautpflaster wird auf die Hydrogel enthaltende Schicht vorzugsweise noch eine Schutzschicht aufgebracht, die vor dem Aufbringen auf die Haut wieder abgezogen wird. Die Schutzschicht ist allgemein eine dünne Polymerfolie, beispielsweise eine Polyethylen-Folie.

Verpackt wird das erfindungsgemäße Pflaster zweckmäßig in einer Folienverpackung, die ein vorzeitiges Austrocknen verhindert.

Die nachfolgenden Beispiele zeigen die Wirksamkeit des erfindungsgemäßen Pflasters zur Behandlung der Neurodermatitis. Insbesondere zeigen sie, dass der Juckreiz für bis zu 2 Stunden, vorzugsweise bis zu 30 Minuten aber mindestens 15 Minuten effektiv gelindert werden konnte. Weiterhin wurde beobachtet, dass die Verfärbung der von der Neurodermatitis betroffenen Hautpartien abgenommen hatte; der Juckreiz wurde effektiv gelindert. Durchgeführt wurden die Tests an 4 Neurodermitis-Patienten. Die Zahlenwerte entsprechen den Zeiträumen einer Temperatursenkung um bis zu 2 Grad Celsius an der Hautoberfläche.

Die nachfolgenden Beispiele dienen zur Illustration der Erfindung. Prozente sind darin als Gewichtsprozente zu verstehen, soweit nicht anders angegeben oder aus dem Zusammenhang unmittelbar ersichtlich.

### Beispiel 1 (ohne Rückschicht):

In einem 250 ml Becherglas wurde eine Mischung aus 18,60 g Glycerin (86 %ig) und 3,70 g Propan-1,2-diol vorgelegt. 3,00 g Carboxymethylcellulose-Na-Salz (®Blanose 7LF) wurden mit einem Spatel eingerührt. 0,12 g Aluminium-glycinat und 0,08 g Na EDTA wurden anschließend zugegeben und die Mischung homogenisiert.

4,30 g Natrium-polyacrylat (®Aronvis MX von Toagosei Co., Ltd.) und 2,20 g einer vernetzten Polycacrylsäure (Carbopol 974P NF von Lubrizol Corp., USA) wurden miteinander vermischt, unter Rühren zu der vorstehend beschriebenen Mischung gegeben und homogenisiert. Anschließend wurden 20,00 g Wasser portionsweise hinzugegeben und verrührt. Die Mischung wurde dabei auf 70 °C erwärmt.

1,00 g Kaolin wurde mit 16,41 g Sorbit (70 %ig, nicht kristallisierend) verrührt, zu der Mischung gegeben und diese dann bis zur optischen Homogenität gerührt.

5,00 g Harnstoff wurde in 10,29 g Wasser gelöst und die Lösung mit 0,50 g eines Konservierungsmittels auf Basis einer Mischung von Phenoxyethanol und 4-Hydroxy-benzoesäure-(C1-C4)alkylester (®Euxyl K300 von Schülke & Mayr GmbH, DE) versetzt. Die Lösung wurde dann unter Rühren zu der vorstehend beschriebenen Mischung gegeben und diese homogenisiert.

1,00 g Dulcamarae stipidites-Extrakt, 1,00 g Hamamelis-Extrakt und 0,50 g Nachtkerzenöl wurden dann hinzugefügt und die Mischung bis zu optischen Homogenität gerührt.

Als nächstes wurden 4,50 g Gelatine unter Rühren zugegeben. Dann wurde eine Lösung aus 0,40 g Weinsäure und 7,00 g Wasser unter Rühren zugegeben und die Mischung mit den oben genannten Wirkstoffen erneut bis zur optischen Homogenität gerührt.

Die Mischung wurde auf einer Glasplatte zu einer Schicht mit einem Gewicht von 700 g/m² aufgetragen und bei 50 °C 120 min lang getrocknet. Die Hydrogelschicht wurde dann von der Glasplatte abgelöst und sofort als Pflaster verwendet.

### Beispiel 2 (ohne Rückschicht):

22,82 g Glycerin (86 %ig) wurde in einem 250 ml-Becherglas vorgelegt. 3,00 g Carboxymethylcellulose-Na-Salz (®Blanose 7 LF) wurden in das Glycerin eingerührt. Anschließend wurde 0,40 g Aluminiumhydroxid-Gel und 0,08 g Na- EDTA vermischt und unter Rühren zu der Vorlage gegeben. Die Mischung wurde dann homogenisiert.

Eine Mischung aus 4,00 g einer teilweise neutralisierten Polyacrylsäure (®Viscomate NP-600 von Showa Denko K.K.) und 2,20 g Polyacrylsäure (®Carbopol 974P NF) wurde dann unter Rühren zu der Vorlage gegeben und die entstehende Mischung homogenisiert. Sodann wurden 20 g Wasser portionsweise zugefügt und verrührt. Die Masse wurde bei 70 °C bis zur optischen Homogenität gerührt.

1,00 g Kaolin, verrührt in 16,40 g Sorbit (70 %ig, nicht kristallisierend), wurden dann in die vorstehend beschriebene Masse eingerührt und homogenisiert.

5,00 g Harnstoff wurden in 10,20 g Wasser gelöst. 0,50 g eines Konservierungsmittels (®Lipoconserv) wurden der Lösung hinzugefügt. Die Lösung wurde dann zu der Vorlage gegeben und homogenisiert.

Eine Wirkstoff-Mischung aus 1,00 g Kamillen-Extrakt, 1,00 g Echinacea-Extrakt und 0,50 g Arganöl wurde dann zugegeben und die entstehende Masse bis zur optischen Homogenität gerührt

Danach wurden 4,50 g Gelatine unter Rühren zugefügt. Dann wurde eine Lösung aus 0,40 g Weinsäure und 7,00 g Wasser unter Rühren zugegeben und die Mischung mit den oben genannten Wirkstoffen erneut bis zur optischen Homogenität gerührt.

Die Mischung wurde wie in Beispiel 1 beschrieben zu einem Pflaster ohne Rückschicht verarbeitet.

### Beispiel 3 (mit Rückschicht aus einem Viskose-Vlies):

In einem 250 ml Becherglas wurde eine Mischung aus 17,85 g Glycerin (86 %ig), 0,30 g eines PEG 8-Caprylsäure/Caprinsäure-Glycerids (®Acconon CC 400) und 3,35 g Polyethylenglykol-400 (®Rotipuran 400) vorgelegt.

0,50 g Carboxymethylcellulose-Na-Salz (®Blanose 7LF) wurden mit einem Spatel eingerührt. 1,30 g Aluminium-glycinat und 2,27 g Na-EDTA wurden anschließend zugegeben und die Mischung homogenisiert.

4,53 g Natrium-polyacrylat (®Aronvis MX von Toagosei Co., Ltd.), 9,00 g eines vernetzten Polycacrylats (Carbopol 934 von Lubrizol Corp., USA) 4,00 g eines Methylmethacrylat/2-Ethyl-hexyl-acrylat-Copolymers und 2,00 g Polyacrylsäure wurden miteinander vermischt, unter Rühren zu der vorstehend beschriebenen Mischung gegeben und homogenisiert. Anschließend wurden 10,00 g Wasser portionsweise hinzugegeben und verrührt bis eine optische homogene Masse entstanden war. Die Mischung wurde dabei auf 70 °C erwärmt.

1,00 g Titandioxid wurde mit 16,40 g Sorbit (70 %ig, nicht kristallisierend) verrührt, zu der Mischung gegeben und diese dann bis zur optischen Homogenität gerührt.

5,00 g Harnstoff wurde in 10,00 g Wasser gelöst und die Lösung mit 0,10 g 4-Hydroxy-benzoesäure-methylester und 0,05 g 4-Hydroxy-benzoesäure-propylester als Konservierungsmittel versetzt. Die Lösung wurde dann unter Rühren zu der vorstehend beschriebenen Mischung gegeben und diese homogenisiert.

1,00 g Kamillen-Extrakt, 1,00 g Echinacea-Extrakt und 0,50 g Arganöl wurden dann hinzugefügt und die Mischung bis zu optischen Homogenität gerührt.

Dann wurde eine Lösung aus 2,85 g Weinsäure und 7,00 g Wasser unter Rühren zugegeben und die Mischung mit den oben genannten Wirkstoffen erneut bis zur optischen Homogenität gerührt.

Die Mischung wurde wie in Beispiel 1 beschrieben auf einer Glasplatte zu einer Schicht mit einem Gewicht von 700 g/m² aufgetragen, bei 50 °C 120 min lang getrocknet und dann - nach Ablösung von der Glasplatte - mit einem Polyestervlies zu einem Pflaster laminiert.

Die Pflaster wurden den vier Probanden (s.o.) auf die Unterarme appliziert und die Hauttemperatur in Abhängigkeit von der Zeit gemessen. Als Vergleich wurden zum einen zwei Hydrogelpatches der Fa. SCS Skin Care Systems GmbH herangezogen (Gel de L'eau, Relax Patch und Gel de l'eau Eye Patch); zum anderen wurde Fenestil® Gel (Novartis, Deutschland) und Pferdesalbe als topische Applikationen zum Vergleich herangezogen. Gegenüber allen Vergleichspflastern bzw. Salben zeigte sich bei den erfindungsgemäßen Hydrogelpflastern eine deutliche Temperaturerniedrigung auf der Haut und damit eine spürbare Erleichterung für die Probanden.

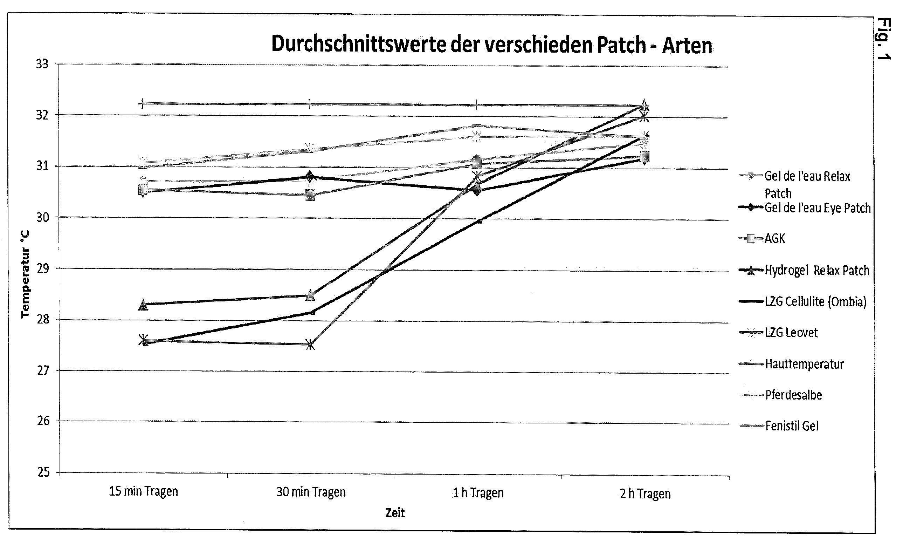

Die Ergebnisse sind in den Tabellen 1 und 2 sowie in Figur 1 zusammengefasst.

**Tabelle 1:**

| Durchschnittswerte aller 4 Testpersonen | | | | | | | |
|---|---|---|---|---|---|---|---|
| **Zeit** | Gel de l'eau | Gel de l'eau | AGK | Hydrogel | LZG | LZG | Hauttemperatur |
| | Relax Patch | Eye Patch | | Relax Patch | Cellulite (Ombia) | Leovet | |
| | | | | | | | |
| 15 min Tragen | 30,7 | 30,5 | 30,55 | 28,3 | 27,525 | 27,6 | 32,225 |
| 30 min Tragen | 30,725 | 30,8 | 30,45 | 28,5 | 28,15 | 27,525 | 32,225 |
| 1 h Tragen | 31,15 | 30,55 | 31,075 | 30,675 | 29,95 | 30,825 | 32,225 |
| 2 h Tragen | 31,475 | 31,175 | 31,225 | 32,25 | 31,625 | 32,025 | 32,225 |

**Tabelle 2:**

| Durchschnittswerte aller 4 Testpersonen | | |
|---|---|---|
| **Mittelwerte** | Pferdesalbe | Fenistil Gel |
| | | |
| Ausgangstemperatur | 31,825 | 31,825 |
| 15 min Tragen | 31,075 | 30,975 |
| 30 min Tragen | 31,35 | 31,3 |
| 1 h Tragen | 31,6 | 31,825 |
| 2 h Tragen | 31,625 | 31,6 |

## Patentansprüche

1. Verwendung eines für Wasserdampf durchlässigen Hautpflasters, das ein Hydrogel, ein Feuchthaltemittel und mindestens einen pharmazeutisch und/oder kosmetisch wirksamen Stoff enthält, zur Behandlung von entzündlichen Erkrankungen der Haut, die in den Gruppen L20 bis L30 der ICD-10 (WHO-Version 2006) aufgeführt sind.

2. Verwendung des Hautpflasters gemäß Anspruch 1 zur Behandlung von Neurodermitis.

3. Verwendung gemäß Anspruch 1 oder 2, **dadurch gekennzeichnet, dass** das Hydrogel aus hydrophilen und in Wasser quellbaren natürlichen und/oder synthetischen Polymeren besteht.

4. Verwendung gemäß Anspruch 3, **dadurch gekennzeichnet, dass** die synthetischen Polymere Polyvinylalkohol, teilverseifte Polyvinylacetate, Polyethylenoxide, Poly(hydroxyalkyl(meth)acrylate) und Polyvinylpyrrolidone sind.

5. Verwendung gemäß Anspruch 3, **dadurch gekennzeichnet, dass** die natürlichen Polymere Carrageenan, Kollagen, Gelatine, Agar-Agar, Alginat und Chitosane sind.

6. Verwendung gemäß einem oder mehreren der Ansprüche 1 bis 5, **dadurch gekennzeichnet, dass** Feuchthaltemittel ausgewählt ist aus Glycerin, Glykolderivaten wie Butylenglykol, PEG, Sorbit, Harnstoff, Cyclomethicone, Dimethicone, Hyaluronsäure/Milchsäure, Aminosäuren und hydrolysierten Proteinen und Zucker.

7. Verwendung gemäß einem oder mehreren der Ansprüche 1 bis 6, **dadurch gekennzeichnet, dass** der Anteil des Feuchthaltemittels am Gesamtgewicht der Hydrogel-enthaltenden Schicht von 0 bis 25 Gew.-% beträgt, bezogen auf das Gesamtgewicht der Hydrogel enthaltenden Schicht.

8. Verwendung gemäß einem oder mehreren der Ansprüche 1 bis 7, **dadurch gekennzeichnet, dass** das pharmazeutisch und/oder kosmetisch wirksame Mittel ein Calcineurin-Inhibitor auf Basis von Makroliden, Cortison, ein pflanzliches Präparate aus der Gruppe der Nachtschattengewächse, oder einem oder mehreren pflanzlichen Präparaten aus Schachtelhalm (Equisetum spp.), Aloe vera, Minze, Bitterorangen, Echinacea, Kamille, Hamamelis virginiana, Schwarzkümmel (Nigella sativa), Viola tricolor, Viola avensis, Johanniskraut (hypericum perforatum), Nachtkerze (oenothera biennis), Calendula (dulcamaris solanis), ist.

9. Verwendung gemäß einem oder mehreren der Ansprüche 1 bis 8, **dadurch gekennzeichnet, dass** das Hautpflaster eine Rückschicht aufweist.

10. Verwendung gemäß Anspruch 9, **dadurch gekennzeichnet, dass** die Rückschicht aus einem Vlies, Gewebe, Gewirke oder einer wasserdampfdurchlässigen Folie besteht.

11. Verwendung gemäß Anspruch 9 oder 10, **dadurch gekennzeichnet, dass** das Gewicht der Rückschicht im Bereich von 70 bis 120 g/m² liegt.

12. Verwendung gemäß einem oder mehreren der Ansprüche 1 bis 11, **dadurch gekennzeichnet, dass** der Schälwiderstand beim Abschälen des Pflasters von der Haut im Bereich von 0,5 bis 4,0 N/25 mm beim 180° Peel-Adhesion-Test liegt.
